# EUROPÄISCHE PATENTSCHRIFT

(11) **EP 1 165 520 B1**
(45) Veröffentlichungstag und Bekanntmachung des Hinweises auf die Patenterteilung: **20.05.2009**
(21) Anmeldenummer: 00912664.0
(22) Anmeldetag: 30.03.2000
(51) Int. Cl.: C07D 237/22

(54) **VERFAHREN ZUR HERSTELLUNG VON 4-AMINO-5-CHLOR-1-PHENYLPYRIDAZINON**
METHOD FOR PRODUCING 4-AMINO-5-CHLORO-1-PHENYL PYRIDAZINONE
PROCEDE DE PREPARATION DE LA 4-AMINO-5-CHLORO-1-PHENYLPYRIDAZINONE

(30) Priorität: 31.03.1999 DE 19914722
(43) Veröffentlichungstag der Anmeldung: 02.01.2002
(73) Patentinhaber: BASF SE, 67056 Ludwigshafen (DE)
(72) Erfinder: MERKLE, Hans, Rupert, D-67061 Ludwigshafen (DE); HERBIG, Klaus, D-67105 Schifferstadt (DE); FRETSCHNER, Erich, D-69239 Neckarsteinach (DE); FRÖHLICH, Helmut, D-67071 Ludwigshafen (DE)
(74) Vertreter: Kinzebach, Werner
(86) Internationale Anmeldenummer: PCT/EP2000/002827
(87) Internationale Veröffentlichungsnummer: WO 2000/059891

(56) Entgegenhaltungen:
- EP-A- 0 028 359

## Beschreibung

Die vorliegende Erfindung betrifft ein Verfahren zur Herstellung von 4-Amino-5-chlor-1-phenylpyridazinon-(6) durch Umsetzung von 4,5-Dichlor-1-phenylpyridazinon-(6) mit wässrigem Ammoniak in Gegenwart eines Katalysators.

4-Amino-5-chlor-1-phenylpyridazinon-(6) (Chloridazon) wird als Herbizid zur selektiven Bekämpfung von Unkräutern in Zuckerrüben verwendet. Die Herstellung dieser Verbindung erfolgt gemäß GB 871674 durch Umsetzung von 4,5-Dichlor-1-phenylpyridazinon-(6) unter Druck mit wässrigem Ammoniak und bei erhöhter Temperatur. Man erhält dabei ein Isomerengemisch von etwa 80 Gew.-% 4-Amino-5-chlor-1-phenylpyridazinon-(6) und etwa 20 Gew.-% 5-Amino-4-chlor-1-phenylpyridazinon-(6). Die Gewinnung des Chloridazons aus diesem Isomerengemisch durch Extraktion des ungewünschten Isomers mit unpolaren Lösungsmitteln ist beispielsweise in der DE 16 20 186 beschrieben.

Außerdem hat man versucht, die Umsetzung so durchzuführen, dass ein reineres Produkt erhalten wird. So beschreibt die DD 131172 die obige Umsetzung in organischen Lösungsmitteln, wobei ein reineres Produkt erhalten wird. Die Verwendung organischer Lösungsmittel anstelle von Wasser ist jedoch nachteilig. In der EP 26 847 A und EP 28 359 A ist die Umsetzung von 4,5-Dichlor-1-phenylpyridazinon-(6) mit wässrigem Ammoniak unter Druck in Anwesenheit von phenolischen Verbindungen beschrieben, die Substituenten aufweisen, welche diese Verbindungen in dem wässrigen Reaktionsmedium löslich machen. Derartige Verbindungen sind beispielsweise 4-Phenolsulfonsäure, 3-Hydroxypyridon und 3-Hydroxypyridin. Man erhält auf diese Weise sehr gute Ausbeuten an Chloridazon hoher Reinheit. Eine Wiederverwendung der eingesetzten Katalysatoren in Form der Lösung in dem Reaktionsmedium ist nicht möglich, weil damit eine Rückführung des bei der Reaktion anfallenden Ammoniumchlorids verbunden wäre. Eine Gewinnung der Katalysatoren aus dem wässrigen Reaktionsmedium ist dagegen aufgrund ihrer hohen Wasserlöslichkeit selbst durch Extraktion mit organischen Lösungsmitteln nur sehr begrenzt möglich.

Der vorliegenden Erfindung liegt daher die Aufgabe zugrunde, ein Verfahren zur Herstellung von Chloridazon zur Verfügung zu stellen, bei dem das Chloridazon in hoher Ausbeute und hoher Reinheit erhalten wird und der verwendete Katalysator in einfacher Weise wiedereingesetzt werden kann.

Überraschenderweise wurde nun gefunden, dass diese Aufgabe durch ein Verfahren gelöst wird, bei dem 4,5-Dichlor-1-phenylpyridazinon-(6) mit wässrigem Ammoniak in Gegenwart eines Katalysators umgesetzt wird, der in dem verwendeten Reaktionsmedium löslich, bei saurem pH in dem Reaktionsmedium jedoch im Wesentlichen unlöslich ist.

Die vorliegende Erfindung betrifft daher ein Verfahren zur Herstellung von 4-Amino-5-chlor-1-phenylpyridazinon-(6) durch Umsetzung von 4,5-Dichlor-1-phenylpyridazinon-(6) mit wässrigem Ammoniak in Gegenwart eines Katalysators, der in dem verwendeten wässrigen Reaktionsmedium (d. h. bei alkalischem pH-Wert) löslich, in dem nach Entfernen des ausgefallenen 4-Amino-5-chlor-1-phenylpyridazinons-(6) sauer eingestellten Reaktionsmedium jedoch im Wesentlichen unlöslich ist.

Der Ammoniak wird für die Umsetzung in großem Überschuss eingesetzt. Der pH-Wert des wässrigen Reaktionsmediums liegt somit im alkalischen Bereich. Die hier verwendeten Katalysatoren sind in diesem Reaktionsmedium löslich.

Gegebenenfalls wird der überschüssige Ammoniak nach der Umsetzung entfernt, z. B. durch Abstrippen vor oder bei dem Abkühlen des Reaktionsgemisches.

Das erhaltene Chloridazon fällt nach der Umsetzung, insbesondere bei Abkühlen des Reaktionsgemisches, als Feststoff aus. Es wird in üblicher Weise aus dem Reaktionsmedium gewonnen, beispielsweise durch Abfiltrieren. Das nach Gewinnung des Chloridazons verbleibende Reaktionsmedium (Mutterlauge) weist alkalischen pH auf. Durch Einstellung eines sauren pH-Wertes, beispielsweise pH < 6, insbesondere < 4 und besonders bevorzugt < 2, mit Hilfe einer anorganischen Säure, wie Salzsäure, Schwefelsäure, Phosphorsäure, oder einer organischen Säure, wie Ameisensäure oder Essigsäure, wird der Katalysator in dem Reaktionsmedium unlöslich. Im Allgemeinen fällt er dabei als amorpher oder kristalliner Feststoff aus und kann somit auf einfache weise gewonnen werden, beispielsweise durch Filtration. Unter "unlöslich in dem Reaktionsmedium" ist hier zu verstehen, dass der Katalysator eine Löslichkeit in dem Reaktionsmedium von höchstens 5 g/l, insbesondere höchstens 2 g/l, aufweist.

Geeignete Katalysatoren sind insbesondere phenolische Verbindungen, deren aromatischer Kern neben der phenolischen Hydroxygruppe mindestens einen weiteren elektronenziehenden Substituenten ohne saure Wasserstoffatome aufweist. Unter phenolischen Verbindungen sind hier Verbindungen mit einem aromatischen carbocyclischen oder heterocyclischen Kern (insbesondere Phenyl, Naphthyl, Pyridyl, Pyrimidyl etc.), der mindestens eine Hydroxygruppe aufweist, zu verstehen.

Der aromatische Kern weist im Allgemeinen einen oder zwei elektronenziehende Substituenten auf. Dabei handelt es sich insbesondere um SO₂R¹, NO₂, COR¹, CF₃, CN und OR¹, wobei R¹ für C₁-C₆-Alkyl oder Phenyl, das gegebenenfalls durch 1 oder 2 Hydroxygruppen substituiert ist, steht.

Brauchbare phenolische Verbindungen sind beispielsweise 2-, 3- oder 4-Nitrophenol, 2,4-Dinitrophenol, 4-Benzoylphenol, 4,4'-Dihydroxybenzophenon und insbesondere Bis(hydroxyphenyl)sulfone, wie Bis(4-hydroxyphenyl)sulfon.

Geeignet sind auch die Salze der phenolischen Verbindungen, z. B. die Alkali- oder Erdalkalimetallsalze sowie die Ammoniumsalze.

Die phenolischen Verbindungen sind in der Phenolatform wasserlöslich, in Form der freien Phenole dagegen im Wesentlichen wasserunlöslich. Sie können daher im Wesentlichen quantitativ, zumindest aber zu wenigstens 80 %, insbesondere zu wenigstens 90 % zurückgewonnen und für weitere Umsetzungen verwendet werden. Auf diese Weise erfolgt außerdem eine Abtrennung von dem bei der Umsetzung gebildeten Ammoniumchlorid. Durch Zusatz von Basen, wie Natronlauge, zu dem nach Abtrennung des Katalysators erhaltenen Reaktionsmedium kann der im Ammoniumchlorid gebundene Ammoniak wieder freigesetzt und zurückgewonnen werden.

Die Umsetzung von 4,5-Dichlor-1-phenylpyridazinon-(6) mit Ammoniak kann einstufig oder zweistufig durchgeführt werden. Bei der einstufigen Variante werden die Reaktionspartner und der Katalysator gleichzeitig eingesetzt. Bei der zweistufigen Variante wird zunächst eine Lösung des Katalysators, beispielsweise eine Natrium- oder Ammoniumsalzlösung des Katalysators, mit 4,5-Dichlor-l-phenylpyridazinon-(6) unter Substitution des Chloratoms in 4-Position durch das entsprechende Phenolat zu dem entsprechenden Zwischenprodukt umgesetzt. Beispielsweise erhält man bei Verwendung von Bis(4-hydroxyphenyl)sulfon als Katalysator das 4-(4-Hydroxyphenylsulfonyl)phenyloxy-5-chlor-1-phenylpyridazinon-(6) als Zwischenprodukt. Das jeweilige Zwischenprodukt wird anschließend mit wässrigem Ammoniak unter Druck in das gewünschte Chloridazon überführt.

Das erfindungsgemäße Verfahren wird im Allgemeinen bei Temperaturen im Bereich von 80 bis 200 °C, insbesondere 100 bis 150 °C und bevorzugt 100 bis 140 °C durchgeführt.

Die zur Anwendung kommende Menge an Katalysator kann in einem weiten Bereich variieren. Zweckmäßigerweise erfolgt die Umsetzung in Gegenwart von 1 bis 200 mol-%, insbesondere 20 bis 150 mol-% Katalysator, bezogen auf 4,5-Dichlor-1-phenylpyridazinon-(6).

Das Verfahren wird bei einem Druck durchgeführt, der im Allgemeinen im Bereich von 1 bis 50 bar, vorzugsweise 3 bis 20 bar, liegt. Besonders bevorzugt führt man das Verfahren bei dem Druck durch, der sich im geschlossenen Reaktionsgefäß bei der gewählten Reaktionstemperatur einstellt. Es ist aber auch möglich, den Druck während der Umsetzung im geschlossenen Gefäß durch Einpressen von Ammoniak zu erhöhen. Hierbei löst sich ein Teil des eingepressten Ammoniaks in dem wässrigen Reaktionsmedium.

Die nachfolgenden Beispiele erläutern die Erfindung, ohne sie zu begrenzen.

### Beispiel 1

### Herstellung von 4-Amino-5-chlor-1-phenylpyridazinon-(6) (Chloridazon) mit Bis(4-hydroxyphenyl)sulfon

In einem 250 ml-Rührautoklav wurden 100 Teile Wasser, 70 Teile (1,03 Mol) Ammoniak 25%ig, 12 Teile (0,05 Mol) 4,5-Dichlor-1-phenylpyridazinon-(6) (Gehalt 99,7 %) und 12,5 Teile (0,05 Mol) Bis(4-hydroxyphenyl)sulfon 8 Stunden bei 130 °C gerührt. Der Druck stieg stetig auf ca. 5 bar. Nach Rühren über Nacht wurde auf Normaldruck entspannt, wobei der überschüssige Ammoniak gestrippt wurde. Nach Abkühlen auf Raumtemperatur wurde der ausgefallene Feststoff abfiltriert, mit Wasser gewaschen und im Vakuumtrockenschrank bei 50 °C getrocknet.

Man erhielt 10,1 Teile 4-Amino-5-chlor-1-phenylpyridazinon-(6) mit einem Gehalt von 98,8 %; das entspricht einer Ausbeute von 90 % d. Th. Das Filtrat wurde mit 60%iger Schwefelsäure auf pH 1,5 eingestellt, das ausgefallene Bis(4-hydroxyphenyl)sulfon wurde abfiltriert, mit Wasser gewaschen und getrocknet. Man erhielt 12,6 Teile Bis(4-hydroxyphenyl)sulfon mit einem Gehalt von 99,2 %; das entspricht 100 % der eingesetzten Katalysatormenge.

### Beispiel 2

### Herstellung von 4-Amino-5-chlor-1-phenylpyridazinon-(6) (Chloridazon) mit Bis(4-hydroxyphenyl)sulfon als Katalysator

In einem 1 1-Rührautoklav wurden 300 Teile Wasser, 210 Teile (3,09 Mol) Ammoniak 25 %ig, 36 Teile (0,15 Mol) 4,5-Dichlor-1-phenylpyridazinon-(6) (Gehalt 99,7 %) und 37,5 Teile (0,15 Mol) Bis(4-hydroxyphenyl)sulfon 8 Stunden bei 130 °C gerührt. Der Druck stieg stetig auf ca. 5 bar. Nach Rühren über Nacht wurde auf Normaldruck entspannt, wobei der überschüssige Ammoniak gestrippt wurde. Nach Abkühlen auf Raumtemperatur wurde der ausgefallene Feststoff abfiltriert, mit Wasser gewaschen und im Vakuumtrockenschrank bei 50 °C getrocknet.

Man erhielt 30,7 Teile 4-Amino-5-chlor-1-phenylpyridazinon-(6) mit einem Gehalt von 99,9 %; das entspricht einer Ausbeute von 92,3 % d. Th. Das Filtrat wurde mit 60%iger Schwefelsäure auf pH 1,4 eingestellt, das ausgefallene Bis(4-hydroxyphenyl)sulfon wurde abfiltriert und mit Wasser gewaschen. Man erhielt 53,5 Teile Bis(4-hydroxyphenyl)sulfon mit einem Wassergehalt von 30 %. Das entspricht 99,8 % der eingesetzten Katalysatormenge.

### Beispiel 3

### Herstellung von 4-Amino-5-chlor-1-phenylpyridazinon-(6) mit rückgeführtem Bis(4-hydroxyphenyl)sulfon

Der feuchte Katalysator aus Beispiel 2 wurde mit 300 Teilen Wasser, 210 Teilen (3,09 Mol) Ammoniak 25%ig und 36 Teilen (0,15 Mol) 4,5-Dichlor-1-phenylpyridazinon-(6) (Gehalt 99,7 %) im 1 1-Rührautoklav 8 Stunden bei 130 °C gerührt. Der Druck stellte sich auf 6 bar ein. Nach Rühren über Nacht wurde auf Normaldruck entspannt, wobei der überschüssige Ammoniak gestrippt wurde. Nach Abkühlen auf Raumtemperatur wurde der ausgefallene Feststoff abfiltriert, mit Wasser gewaschen und im Vakuumtrockenschrank bei 50 °C getrocknet.

Man erhielt 30,5 Teile 4-Amino-5-chlor-1-phenylpyridazinon-(6) mit einem Gehalt von 99,5 %; das entspricht einer Ausbeute von 91,3 % d. Th. Das Filtrat wurde mit 60%iger Schwefelsäure auf pH 1,4 eingestellt, das ausgefallene Bis(4-hydroxyphenyl)sulfon wurde abfiltriert, und mit Wasser gewaschen und getrocknet. Man erhielt 37,1 Teile Bis(4-hydroxyphenyl)sulfon mit einem Gehalt von 99,5 %; das entspricht 98,4 % der eingesetzten Katalysatormenge.

### Beispiel 4

### Herstellung von 4-Amino-5-chlor-1-phenylpyridazinon_-(6) (Chloridazon) mit 4-Nitrophenol als Katalysator

In einem 250 ml-Rührautoklav wurden 100 Teile Wasser, 70 Teile (1,03 Mol) Ammoniak 25%ig, 12 Teile (0,05 Mol) 4,5-Dichlor-1-phenylpyridazinon-(6) (Gehalt 99,7 %) und 14,2 Teile (0,1 Mol) 4-Nitrophenol 98 %ig 8 Stunden bei 130 °C gerührt. Der Druck stieg auf ca. 4 bar. Nach Rühren über Nacht wurde auf Normaldruck entspannt, wobei der überschüssige Ammoniak gestrippt wurde. Nach Abkühlen auf Raumtemperatur wurde der ausgefallene Feststoff abfiltriert, mit Wasser gewaschen und im Vakuumtrockenschrank bei 50 °C getrocknet.

Man erhielt 10,3 Teile 4-Amino-5-chlor-1-phenylpyridazinon-(6) mit einem Gehalt von 99,1 %; das entspricht einer Ausbeute von 92,2 % d. Th. Das Filtrat wurde mit 60%iger Schwefelsäure auf pH 1,0 eingestellt, das ausgefallene 4-Nitrophenol wurde abfiltriert, mit Wasser gewaschen und getrocknet. Man erhielt 14 Teile 4-Nitrophenol mit einem Gehalt von 98,1 %; das entspricht 98,8 % der eingesetzten Katalysatormenge.

### Beispiel 5

### Herstellung von 4-Amino-chlor-1-phenylpyridazinon-(6) (Chloridazon) mit 4,4'-Dihydroxybenzophenon als Katalysator

In einem 250 ml-Rührautoklav wurden 100 Teile Wasser, 70 Teile (1,03 Mol) Ammoniak 25%ig, 12 Teile (0,05 Mol) 4,5-Dichlor-1-phenylpyridazinon-(6) (Gehalt 99,7 %) und 10,7 Teile (0,05 Mol) 4,4_{'}-Dihydroxybenzophenon 8 Stunden bei 130 °C gerührt. Der Druck stieg stetig auf ca. 4,5 bar. Nach Rühren über Nacht wurde auf Normaldruck entspannt, wobei der überschüssige Ammoniak gestrippt wurde. Nach Abkühlen auf Raumtemperatur wurde der ausgefallene Feststoff abfiltriert, mit Wasser gewaschen und im Vakuumtrockenschrank bei 50 °C getrocknet.

Man erhielt 10,2 Teile 4-Amino-5-chlor-1-phenylpyridazinon-(6) mit einem Gehalt von 98,9 %; das entspricht einer Ausbeute von 91,1 % d. Th. Das Filtrat wurde mit 60%iger Schwefelsäure auf pH 1,5 eingestellt, das ausgefallene 4,4'-Dihydroxybenzophenon wurde abfiltriert, mit Wasser gewaschen und getrocknet. Man erhielt 10,9 Teile 4,4'-Dihydroxybenzophenon mit einem Gehalt von 98,3 %; das entspricht 100 % der eingesetzten Katalysatormenge.

### Beispiel 6

### Herstellung von 4-Amino-chlor-1-phenylpyridazinon-(6) (Chloridazon) mit rückgeführtem 4,4'-Dihydroxybenzophenon als Katalysator

Der Katalysator aus Beispiel 5 wurde mit 100 Teilen Wasser, 70 Teilen (1,03 Mol) Ammoniak 25%ig, 12 Teilen (0,05 Mol) 4,5-Dichlor-1-phenylpyridazinon-(6) (Gehalt 99,7 %) in einem 250 ml-Rührautoklav 8 Stunden bei 130 °C gerührt. Der Druck stellte sich auf ca. 4,3 bar ein. Nach Rühren über Nacht wurde auf Normaldruck entspannt, wobei der überschüssige Ammoniak gestrippt wurde. Nach Abkühlen auf Raumtemperatur wurde der ausgefallene Feststoff abfiltriert, mit Wasser gewaschen und im Vakuumtrockenschrank bei 50 °C getrocknet.

Man erhielt 10 Teile 4-Amino-5-chlor-1-phenylpyridazinon-(6) mit einem Gehalt von 99,1 %; das entspricht einer Ausbeute von 89,5 % d. Th. Das Filtrat wurde mit 60%iger Schwefelsäure auf pH 1,5 eingestellt, das ausgefallene 4,4'-Dihydroxybenzophenon wurde abfiltriert, mit Wasser gewaschen und getrocknet. Man erhielt 10,7 Teile 4,4'-Dihydroxybenzophenon mit einem Gehalt von 99,2 %; das entspricht 99,2 % der eingesetzten Katalysatormenge.

## Patentansprüche

1. Verfahren zur Herstellung von 4-Amino-5-chlor-1-phenylpyridazinon-(6), bei dem man
a) 4,5-Dichlor-1-phenylpyridazinon-(6) mit wässrigem Ammoniak in Gegenwart eines Katalysators umsetzt, der unter Verbindungen mit einem aromatischen carbocyclischen oder heterocyclischen Ring mit wenigstens einer an den Ring gebundenen Hydroxygruppe, wobei der aromatische Ring neben der/den gebundenen Hydroxygruppe(n) mindestens einen weiteren elektronenziehenden Substituenten ohne saure Wasserstoffatome aufweist, der ausgewählt ist unter SO₂R¹, NO₂, COR¹, CF₃ oder CN, wobei R¹ für C₁-C₆-Alkyl oder Phenyl, das gegebenenfalls durch 1 oder 2 Hydroxygruppen substituiert ist, oder einem Salz einer der genannten Verbindungen ausgewählt ist;
b) das ausgefallene 4-Amino-5-chlor-1-phenylpyridazinon-(6) entfernt;
c) das Reaktionsmedium sauer einstellt; und
d) den ausgefallenen Katalysator zurückgewinnt.

2. Verfahren nach Anspruch 1, **dadurch gekennzeichnet, dass** man als Katalysator Bis(4-hydroxyphenyl)sulfon, 2-, 3- oder 4-Nitrophenol oder 2,4-Dinitroptienol verwendet.

3. Verfahren nach einem der vorhergehenden Ansprüche, **dadurch gekennzeichnet, dass** man den Katalysator in einer Menge von 1 bis 200 mol-%, bezogen auf 4,5-Dichlor-1-phenylpyridazinon-(6), verwendet.

4. Verfahren nach einem der vorhergehenden Ansprüche, **dadurch gekennzeichnet, dass** man die Umsetzung bei einer Temperatur im Bereich von 80 bis 200 °C durchführt.

5. Verfahren nach einem der vorhergehenden Ansprüche, **dadurch gekennzeichnet, dass** man die Umsetzung bei einem Druck im Bereich von 1 bis 50 bar durchführt.

## Claims

1. A process for preparing 4-amino-5-chloro-1-phenylpyridazin-6-one by
a) reacting 4,5-dichloro-i-phenylpyridazin-6-one with aqueous ammonia in the presence of a catalyst which is selected from among compounds having an aromatic carbocyclic or heterocyclic ring having at least one hydroxy group bonded to the ring and having, in addition to the bonded hydroxy group(s), at least one further electron-withdrawing substituent without acidic hydrogen atoms which is selected from amongst SO₂R¹, NO₂ COR¹, CF₃ and CN, where R¹ is C₁-C₆-alkyl or phenyl which may be substituted by 1 or 2 hydroxy groups, and salts of the compounds mentioned;
b) removing the precipitated 4-amino-5-chloro-1-phenylpyridazin-6-one;
c) acidifying the reaction medium; and
d) recovering the precipitated catalyst.

2. The process according to claim 1 wherein the catalyst used is bis(4-hydroxyphenyl) sulfone, 2-, 3- or 4-nitrophenol or 2,4-dinitrophenol.

3. The process according to any of the preceding claims, wherein the catalyst is used in an amount of from 1 to 200 mol%, based on 4,5-dichloro-1-phenylpyridazin-6-one.

4. The process according to any of the preceding claims, wherein the reaction is carried out at a temperature in the range from 80 to 200°C.

5. The process according to any of the preceding claims, wherein the reaction is carried out at a pressure in the range from 1 to 50 bar.

## Revendications

1. Procédé pour la préparation de 4-amino-5-chloro-1-phénylpyridazinone-(6), dans lequel
a) on fait réagir de la 4,5-dichloro-1-phénylpyridazinone-(6) avec de l'ammoniaque en présence d'un catalyseur qui est choisi parmi des composés comportant un cycle aromatique carbocyclique ou hétérocyclique et comportant au moins un groupe hydroxy lié au cycle, le cycle aromatique comportant en plus du/des groupe(s) hydroxy lié(s) au moins un autre substituant attirant les électrons, sans atomes d'hydrogène acides, qui est choisi parmi SO₂R¹, NO₂, COR¹, CF₃ ou CN, R¹ représentant un groupe alkyle en C₁-C₆ ou phényle, qui est éventuellement substitué par 1 ou 2 groupes hydroxy, ou un sel de l'un des composés nommés ;
b) on sépare la 4-amino-5-chloro-1-phénylpyridazone-(6) précipitée ;
c) on acidifie le milieu réactionnel ; et
d) on récupère le catalyseur précipité.

2. Procédé selon la revendication 1, **caractérisée en ce qu'**on utilise comme catalyseur de la bis(4-hydroxyphényl)sulfone, du 2-, 3- ou 4-nitrophénol ou du 2,4-dinitrophénol.

3. Procédé selon l'une quelconque des revendications précédentes, **caractérisé en ce que** le catalyseur est utilisé en une quantité de 1 à 200 % en moles, par rapport à la 4,5-dichloro-1-phénylhydrazinone-(6).

4. Procédé selon l'une quelconque des revendications précédentes, **caractérisé en ce qu'**on effectue la réaction à une température dans la plage de 80 à 200 °C.

5. Procédé selon l'une quelconque des revendications précédentes, **caractérisé en ce qu'**on effectue la réaction sous une pression dans la plage de 1 à 50 bars.
